# EUROPEAN PATENT APPLICATION

(11) **EP 4 589 020 A1**
(43) Date of publication of application: **23.07.2025**
(21) Application number: 22958510.4
(22) Date of filing: 16.09.2022
(51) Int. Cl.: C12Q 1/6869, C12M 1/34, C12M 1/36, C12M 1/00

(54) **GENE SEQUENCING METHOD, APPARATUS AND DEVICE, AND MEDIUM**

(71) Applicant: National Institute for Communicable Disease Control and Prevention, Chinese Center for Disease Control and Prevention, Beijing, China, Beijing 102299 (CN); Wuhan MGI Tech Co., Ltd., Wuhan, Hubei 430000 (CN)
(72) Inventor: JIANG, Yao, Wuhan, Hubei 430000 (CN); LU, Xin, Wuhan, Hubei 430000 (CN); GONG, Meihua, Wuhan, Hubei 430000 (CN); KAN, Biao, Wuhan, Hubei 430000 (CN); LIANG, Xinming, Wuhan, Hubei 430000 (CN); HE, Jiwei, Wuhan, Hubei 430000 (CN); LI, Zhenpeng, Wuhan, Hubei 430000 (CN); TANG, Yue, Wuhan, Hubei 430000 (CN); LIN, Ying, Wuhan, Hubei 430000 (CN); WANG, Le, Wuhan, Hubei 430000 (CN); JIANG, Hui, Wuhan, Hubei 430000 (CN); HUANG, Yong, Wuhan, Hubei 430000 (CN); ZHANG, Li, Wuhan, Hubei 430000 (CN); SHI, Jianwen, Wuhan, Hubei 430000 (CN); SUN, Jing, Wuhan, Hubei 430000 (CN); YU, Zhixue, Wuhan, Hubei 430000 (CN); DONG, Fu, Wuhan, Hubei 430000 (CN); LI, Qian, Wuhan, Hubei 430000 (CN); ZHANG, Xiwen, Wuhan, Hubei 430000 (CN); RAO, Junhua, Wuhan, Hubei 430000 (CN); HUANG, Shunkai, Wuhan, Hubei 430000 (CN)
(74) Representative: Kransell & Wennborg KB
(86) International application number: PCT/CN2022/119453
(87) International publication number: WO 2024/055320

(57) **Abstract**

Provided are a gene sequencing method, apparatus and device, and a medium. The method includes: acquiring a gene sample to be detected and a preset read length; determining a sample type of each sample included in the gene sample; sequencing, for each sample in the gene sample, each short sequence of the sample based on a sequencing sequence corresponding to the sample type of the sample, until the gene sequence in each short sequence of the sample is sequenced to the preset read length, to obtain intermediate stage sequencing result data of each short sequence in the sample; and sending the intermediate stage sequencing result data of each short sequence in each sample to a target server, enabling the target server to perform data analysis on the intermediate stage sequencing result data of each short sequence in each sample, to obtain an intermediate stage detection report.

## Description

### FIELD

The present disclosure belongs to the technical field of gene sequencing, and particularly, relates to a gene sequencing method, apparatus and device, and a medium.

### BACKGROUND

Gene sequencing is a novel gene detection technology, capable of analyzing and determining the complete sequence of genes derived from blood or saliva as well as predicting the possibility of suffering from various diseases, individual behavior characteristics and rational behavior.

**In** the existing sequencing process, single-end and paired-end sequencing both take a long period of time, for example, requiring more than 24 hours for PE100 sequencing and about 12 hours for SE100 sequencing. Thus, customers have to wait for a long period of time to obtain the report results, resulting in poor customer experience.

### SUMMARY

The present disclosure provides a gene sequencing method, apparatus and device, and a medium, to solve the technical problems in the art, i.e., taking a long time to obtain the report results and a poor customer experience, which are attributed the barcode label sequencing performed at the end of sequencing cycles.

In order to achieve the above objective, the present disclosure provides the following technical solutions.

In a first aspect, the present disclosure provides a gene sequencing method. The gene sequencing method includes:
acquiring a gene sample to be detected and a preset read length, the gene sample including at least one sample, any one sample of the at least one sample including at least one short sequence, each short sequence including a gene sequence to be detected and at most two barcode labels, and in the case that a short sequence includes at least one barcode label, the at least one barcode label in the short sequence being located upstream of the gene sequence;
determining a sample type of each of the at least one sample included in the gene sample;
sequencing, for each of the at least one sample in the gene sample, each short sequence of the sample based on a sequencing sequence corresponding to the sample type of the sample, until the gene sequence in each short sequence of the sample is sequenced to the preset read length, to obtain intermediate stage sequencing result data of each short sequence in the sample; and
sending, to a target server, the intermediate stage sequencing result data of each short sequence in each of the at least one sample, and performing, by the target server, data analysis on the intermediate stage sequencing result data of each short sequence in each of the at least one sample, to obtain an intermediate stage detection report.

In a first possible implementation of the first aspect, said determining the sample type of each of the at least one sample included in the gene sample includes:
in the case that the gene sample comprises one sample, determining that the one sample belongs to a barcode-absent single sample type; and
in the case that the gene sample comprises a plurality of samples, for each sample of the plurality of samples,
   determining, when a short sequence of the sample comprises one barcode label, that the sample belongs to a single-barcode multi-sample type;
   determining, when the short sequence of the sample comprises two barcode labels located in the same strand, that the sample belongs to a one-strand-dual-barcode multi-sample type; and
   determining, when the short sequence of the sample comprises two barcode labels located in two strands, that the sample belongs to a dual-strand-dual-barcode multi-sample type.

In a second possible implementation of the first aspect, a sequencing sequence corresponding to the barcode-absent single sample type is: sequencing the gene sequence in each short sequence of the sample of the barcode-absent single sample type; a sequencing sequence corresponding to the single-barcode multi-sample type is: sequencing the barcode label in each short sequence of the sample of the single-barcode multi-sample type, and sequencing, subsequent to the completion of said sequencing the barcode label, the gene sequence in each short sequence of the sample of the single-barcode multi-sample type; a sequencing sequence corresponding to the one-strand-dual-barcode multi-sample type is: sequencing the two barcode labels in each short sequence of the sample of the one-strand-dual-barcode multi-sample type, separately, and sequencing, subsequent to the completion of said sequencing the two barcode labels, the gene sequence in each short sequence of the sample of the one-strand-dual-barcode multi-sample type; and a sequencing sequence corresponding to the dual-strand-dual-barcode multi-sample type is: sequencing a first barcode label in each short sequence of the sample of the dual-strand-dual-barcode multi-sample type, sequencing, subsequent to the completion of said sequencing the first barcode label, the gene sequence in each short sequence of the sample of the dual-strand-dual-barcode multi-sample type, and sequencing, subsequent to the completion of said sequencing the gene sequence, a second barcode label in each short sequence of the sample of the dual-strand-dual-barcode multi-sample type.

In a third possible implementation of the first aspect, barcode primers used to sequence each short sequence of any one sample of multi-sample types have a smaller length than historical barcode primers. The multi-sample types include the single-barcode multi-sample type, the one-strand-dual-barcode multi-sample type, and the dual-strand-dual-barcode multi-sample type.

In a fourth possible implementation of the first aspect, the preset read length includes at least one read length.

In a fifth possible implementation of the first aspect, when the gene sample comprises a plurality of samples, said sending, to the target server, the intermediate stage sequencing result data of each short sequence in each of the plurality of samples includes:
splitting and classifying, based on barcode labels corresponding to the plurality of samples, the intermediate stage sequencing result data of each short sequence in the plurality of samples, to obtain the intermediate stage sequencing result data corresponding to the plurality of samples; and
sending the intermediate stage sequencing result data corresponding to the plurality of samples to the target server.

In a sixth possible implementation of the first aspect, the gene sequencing method further includes:
completely sequencing, for each of the at least one sample in the gene sample, each short sequence of the sample based on the sequencing sequence corresponding to the sample type of the sample, to obtain complete sequencing result data of each short sequence in the sample; and
sending, to the target server, the complete sequencing result data of each short sequence in each sample, and performing, by the target server, data analysis on the complete sequencing result data of each short sequence in each sample, to obtain a complete detection report.

In a seventh possible implementation of the first aspect, the intermediate stage detection report includes intermediate stage quality control results and intermediate stage identification results of each sample; the complete detection report includes complete quality control results, complete identification results, complete assembly results, and complete source-tracing results of each sample; the intermediate stage quality control results and the complete quality control results of one sample are used to reflect the short sequences of the one sample that have a higher quality than a preset quality threshold; the intermediate stage identification results and the complete identification results of one sample are used to reflect a pathogen concentration information of the one sample; the complete assembly results of one sample are used to reflect a recombinant sample assembled from all the short sequences of the one sample; and the complete source-tracing results of one sample are used to reflect a subtype of the one sample.

In an eighth possible implementation of the first aspect, when the preset read length comprises a first read length and a second read length greater than the first read length: an intermediate stage detection report at the first read length refers to a detection report obtained by analyzing the intermediate stage sequencing result data of each short sequence of each sample at the first read length; and an intermediate stage detection report at the second read length and the complete detection report both refer to a detection report obtained by analyzing the intermediate stage sequencing result data of short sequences identified as non-host sequences in each sample at the second read length.

In a second aspect, the present disclosure provides a gene sequencing apparatus. The gene sequencing apparatus includes a data acquisition module, a sample type determination module, a first sequencing module, and a sequencing result data sending module. The data acquisition module is configured to acquire a gene sample to be detected and a preset read length. The gene sample includes at least one sample. Any one sample of the at least one sample includes at least one short sequence. Each short sequence includes a gene sequence to be detected and at most two barcode labels. In the case that a short sequence includes at least one barcode label, the at least one barcode label in the short sequence is located upstream of the gene sequence. The sample type determination module is configured to determine a sample type of each of the at least one sample comprised in the gene sample. The first sequencing module is configured to sequence, for each of the at least one sample in the gene sample, each short sequence of the sample based on a sequencing sequence corresponding to the sample type of the sample, until the gene sequence in each short sequence of the sample is sequenced to the preset read length, to obtain intermediate stage sequencing result data of each short sequence in the sample. The sequencing result data sending module is configured to send, to a target server, the intermediate stage sequencing result data of each short sequence in each of the at least one sample, enabling the target server to perform data analysis on the intermediate stage sequencing result data of each short sequence in each of the at least one sample, to obtain an intermediate stage detection report.

In a third aspect, the present disclosure provides a gene sequencing device. The gene sequencing device includes a memory and a processor. The memory has a program stored thereon. The processor is configured to execute the program to implement respective steps of the gene sequencing method as described.

In a fourth aspect, the present disclosure provides readable storage medium having a computer program stored thereon. The computer program, when executed by a processor, implements respective steps of the gene sequencing method as described.

In summary, in the gene sequencing method, apparatus and device, and the medium provided by the present disclosure, when the short sequence of the sample includes barcode labels, at least one barcode label in the short sequence is located upstream of the gene sequence. Accordingly, when each short sequence of the sample is sequenced based on the sequencing sequence corresponding to the sample type of the sample, if the short sequence of the sample includes barcode labels, at least one barcode label located upstream in each short sequence of the sample is first sequenced, and then the gene sequence in each short sequence of the sample is sequenced. Thus, even the gene sample includes a plurality of samples, the present disclosure enables the target server to generate the intermediate stage detection report based on intermediate stage sequencing result data obtained by sequencing to the preset read length. In this way, the preliminary pathogen identification can be performed in advance before the complete sequencing is finished, thereby accelerating the detection speed and shortening the customers' waiting time. Therefore, the customers can have a better experience.

### BRIEF DESCRIPTION OF THE DRAWINGS

In order to clearly illustrate the technical solutions in the embodiments of the present disclosure or the related art, the accompanying drawings required for describing the embodiments or the related art are briefly described below. Apparently, the accompanying drawings in the following description merely illustrate embodiments of the present disclosure. Those skilled in the art can derive other accompanying drawings from the provided accompanying drawings without creative efforts.
FIG. 1 is a schematic flow diagram of a gene sequencing method according to an embodiment of the present disclosure;
FIG. 2 is a schematic diagram of an analysis by sequencing application process according to an embodiment of the present disclosure;
FIG. 3 is a schematic diagram illustrating time periods for sequencing samples of multi-sample types to obtain three stage detection reports;
FIG. 4 is a schematic structural diagram of a gene sequencing apparatus according to an embodiment of the present disclosure; and
FIG. 5 is a block diagram of a hardware architecture of a gene sequencing device according to an embodiment of the present disclosure.

### DETAILED DESCRIPTION

The technical solutions in the embodiments of the present disclosure are clearly and thoroughly described with reference to the accompanying drawings in the embodiments of the present disclosure. The described embodiments are only a part rather than all of the embodiments of the present disclosure. All other embodiments obtained by those skilled in the art based on the embodiments of the present disclosure without creative efforts shall fall within the protection scope of the present disclosure.

At present, when performing gene test, a sample to be detected is first subjected to single-end (SE) sequencing and paired-end (PE) sequencing using a gene sequencer (e.g., Sequencer 200Plus), and barcode labels of the sample to be detected are then sequenced at the end of sequencing cycle. After the sequencing is completed, all the sequencing data is uploaded to a server for data analysis to obtain a complete detection report, and the complete detection report is delivered to the customer.

However, as the sequencing of the barcode labels is performed at the end of sequencing cycle, the data analysis process can only be performed when the complete sequencing is finished, and thus the customers have to wait for a long period of time to obtain the report results, resulting in poor customer experience.

In order to solve the problems in the related art, the present disclosure provides a gene sequencing method, apparatus and device, and a medium. Optionally, the gene sequencing method, apparatus and device, and the medium can be applied to a gene sequencer. The gene sequencing method provided in the present disclosure will be first described in detail through the following embodiments.

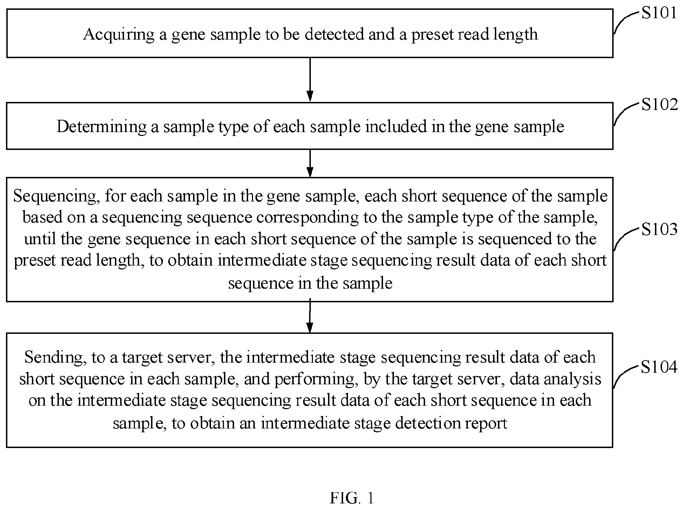

FIG. 1 is a schematic flow diagram of a gene sequencing method according to an embodiment of the present disclosure is shown. With reference to FIG. 1, the gene sequencing method may include the following steps.

In step S101, a gene sample to be detected and a preset read length are acquired.

In this step, the gene sample to be detected (i.e., a genome to be detected) includes at least one sample.

It can be understood that prior to sequencing the gene sample, each sample included in the gene sample may be broken into long DNA fragments with a relatively great molecular weight, and then the long DNA fragments are assigned to reaction spaces with different label sequences. In each of the different label sequences, short sequences carrying the same barcode label are prepared for sequencing. That is, each sample includes at least one short sequence, which belongs to at least one long DNA fragment.

In the present disclosure, the gene sequence to be detected refers to a gene sequence required to be sequenced, and the barcode label refers to a barcode label serving as identity information of the gene sequence.

In this step, each short sequence includes the gene sequence to be detected and at most two barcode labels. In the case that the short sequence includes the barcode labels, at least one barcode label in the short sequence is located upstream of the gene sequence.

The expression "each short sequence includes at most two barcode labels" means that: if the gene sample includes one sample, it is unnecessary to distinguish the sample by means of barcode labels, and thus each short sequence of the sample can only include the gene sequence to be detected in this case, without carrying the barcode labels; and if the gene sample includes a plurality of samples, it is necessary to distinguish the plurality of samples by means of the barcode labels, and short sequence of each sample includes one or two barcode labels in this case.

In the present embodiment, in the case that a short sequence of a sample includes barcode labels, at least one barcode label in the short sequence is located upstream of the gene sequence in the short sequence. If each short sequence of a sample includes one barcode label, each short sequence of the sample consists of the barcode label and the gene sequence in sequence, i.e., the barcode label is located upstream of the gene sequence. If each short sequence of a sample includes two barcode labels, at least one of the two barcode labels is located upstream of the gene sequence in each short sequence. For example, the two barcode labels are denoted as **barcode1** and **barcode2,** respectively, and each short sequence of the sample can be in the form of **BC1readBC2** or **BC1BC2read,** where the **"BC"** represents barcode and the **"read"** represents the gene sequence.

The preset read length refers to the number of cycles required to generate a report. In the present embodiment, the specific value of the preset read length can be determined depending on actual conditions. For example, in the currently feasible scenarios, for the single-end sequencing (SE) read length, a customer may customize any read length ranging from 1 BP (base pair) to 100 BP at a single end. For example, in the present embodiment, the preset read length is 40 bp (base pair), indicating that an intermediate stage detection report is generated after 40 cycles of sequencing of the gene sequence.

In an optional embodiment, the preset read length includes at least one read length. For example, the preset read lengths are 40 bp and 80 bp, indicating that in the present embodiment, a first intermediate stage detection report is generated when a gene sequence after 40 cycles of sequencing of the gene sequence, and a second intermediate stage detection report is generated after 80 cycles of sequencing of the gene sequence.

It should be noted that the pathogen identification (i.e., the intermediate stage detection report) is more accurate with the increase in the preset read length.

In step S102, a sample type of each sample included in the gene sample is determined.

Optionally, the sample types include, but not limited to, the following four types: a barcode-absent single sample type **NoneBC,** a single-barcode multi-sample type **SingleBC,** a one-strand-dual-barcode multi-sample type **BC1BC2read,** and a dual-strand-dual-barcode multi-sample type **BC1readBC2.**

It should be noted that the terms "single-sample" and "multi-sample" here refer to the number of samples included in a gene sample. If the gene sample includes one sample, the one sample belongs to the single-sample type, and if the gene sample contains a plurality of samples, the plurality of samples belong to the multi-sample type.

It can be understood that when the gene sample contains only one sample, it is unnecessary to distinguish by means of barcode labels, in which case the only sample belongs to a barcode-absent single sample type.

When the gene sample contains a plurality of samples, it is required to distinguish the samples by means of barcode labels. In this case, the short sequence of each sample may contain one barcode label or two barcode labels.

If a short sequence of a sample contains one barcode label, i.e., the short sequence of the sample is obtained by splicing the barcode label and the gene sequence (the barcode label is located upstream of the gene sequence). In this step, it can be determined that the sample belongs to a single-barcode multi-sample type.

If a short sequence of a sample contains two barcode labels and the two barcode labels are located in the same strand, it can be determined in this step that the sample belongs to a one-strand-dual-barcode multi-sample type. For example, if a short sequence of a sample contains **barcode1** and **barcode2** on one strand of the gene sequence (**barcode1** and **barcode2** are located upstream of the gene data on one strand), it can be determined that the sample belongs to the one-strand-dual-barcode multi-sample type.

If a short sequence of a sample contains two barcode labels and the two barcode labels are located in two strands, it can be determined in this step that the sample belongs to a dual-strand-dual-barcode multi-sample type. For example, if a short sequence of a sample contains **barcode1** on one strand (**barcode1** is located upstream of the gene data on one strand) and **barcode2** at the end of the other strand (gene data on the other strand is located upstream of **barcode2**), it can be determined that the sample belongs to the dual-strand-dual-barcode multi-sample type.

In step S103, for each sample in the gene sample, each short sequence of the sample is sequenced based on a sequencing sequence corresponding to the sample type of the sample, until the gene sequence in each short sequence of the sample is sequenced to the preset read length, to obtain intermediate stage sequencing result data of each short sequence in the sample.

In the previous steps, it has been stated that "in the case that a short sequence of a sample includes barcode labels, at least one barcode label in the short sequence is located upstream of the gene sequence in the short sequence". The gene sequencing is the sequential sequencing of each data in the short sequence of the sample. Therefore, when the gene sample is of multi-sample types, according to the sequencing sequence corresponding to any multi-sample type, at least one barcode label in the short sequence of the sample of the multi-sample types is always sequenced first, and thereafter, the gene sequence in the sample of the multi-sample types is sequenced.

Therefore, if the gene sample includes one sample, the intermediate stage sequencing result data includes intermediate stage sequencing result data of the gene sequence, and if the gene sample includes a plurality of samples, the intermediate stage sequencing result data includes intermediate stage sequencing result data of the gene sequence and sequencing result data of the barcode label.

In the present disclosure, the multi-sample types include a single-barcode multi-sample type, a one-strand-dual-barcode multi-sample type, and a dual-strand-dual-barcode multi-sample type.

In step S104, the intermediate stage sequencing result data of each short sequence in each sample is sent to a target server, and the target server performs data analysis on the intermediate stage sequencing result data of each short sequence in each sample to obtain an intermediate stage detection report.

Specifically, if the gene sample includes one sample, the target server directly performs data analysis on the intermediate stage sequencing result data of each short sequence in the one sample to obtain an intermediate stage detection report.

If the gene sample includes a plurality of samples, the target server performs data analysis on the intermediate stage sequencing result data of the gene sequence of each short sequence in the plurality of samples based on the barcode label sequencing result data of the plurality of samples to obtain an intermediate stage detection report. The intermediate stage detection report includes pathogen identification results of read sequences obtained at the preset read length. It can be understood that the pathogen identification results included in the intermediate stage detection report are relatively rough results due to incomplete sequencing. However, relatively accurate pathogen identification results can be obtained if the preset read length is appropriately selected. For example, when the preset read length is 40 bp, the preliminary identification results obtained by data analysis are basically consistent with the identification results of sequencing data obtained with the complete read length (i.e., 100 bp), which also indicates that the intermediate stage detection report obtained at the read length of 40 bp has high accuracy.

In the gene sequencing method provided by the present disclosure, when the short sequence of the sample includes barcode labels, at least one barcode label in the short sequence is located upstream of the gene sequence. Accordingly, when each short sequence of the sample is sequenced based on the sequencing sequence corresponding to the sample type of the sample, if the short sequence of the sample includes barcode labels, at least one barcode label located upstream in each short sequence of the sample is first sequenced, and then the gene sequence in each short sequence of the sample is sequenced. Thus, even the gene sample includes a plurality of samples, the present disclosure enables the target server to generate the intermediate stage detection report based on intermediate stage sequencing result data obtained by sequencing to the preset read length. In this way, the preliminary pathogen identification can be performed in advance before the complete sequencing is finished, thereby accelerating the detection speed and shortening the customers' waiting time. Therefore, the customers can have a better experience.

For example, in a possible scenario, a customer, after submitting a sequencing request, may wish to obtain pathogen identification results in a relatively short period of time, e.g., within 10 hours. However, the current gene sequencing techniques require more than 24 hours to obtain accurate identification results, resulting in a poor customer experience.

In contrast, by adopting the gene sequencing method provided in the present disclosure, a preset read length can be set by the customer according to his or her own requirements. If the customer has sufficient time to wait for the pathogen identification results, one or more greater preset read lengths can be set to obtain more accurate pathogen identification results. If the customer does not have sufficient time to wait for the pathogen identification results, one or more smaller preset read lengths can be set to obtain rough preliminary pathogen identification results in a short period of time.

According to the present embodiment, by customizing the preset read length, partial data can be obtained at an intermediate sequencing stage for analysis, and the customer can be provided with the obtained preliminary report for preliminary screening, thereby shortening the waiting time and improving the customer experience.

In a possible implementation of the present disclosure, the sequencing sequences corresponding to the four sample types provided in step S102 are described.

In the present embodiment, the sequencing sequences corresponding to the four sample types are related with the position of barcode labels and gene sequences included in samples of the four sample types.

Specifically, the sequencing sequence corresponding to the barcode-absent single sample type is to sequence the gene sequence in each short sequence of the sample of the barcode-absent single sample type. Specifically, a single-end (SE) sequencing process includes: DNA nanoball (DNB) loading -> pre-loading (loading prime) -> loading (postloading) -> sequencing pre-treatment (sequence prime) -> sequencing pre-treatment cleaning (first) -> read1 (first DNB) sequencing. A paired-end (PE) sequencing process includes: DNB loading -> loading prime -> postloading -> sequence prime -> sequence prime cleaning -> read1 (first DNB) sequencing -> second strand synthesis (PE synthesis) -> read2 (first DNB) sequencing. The specific process of each of the above sub-processes is the same as that of the related art, which will not be described in detail herein.

Optionally, the sequencing sequence corresponding to the single-barcode multi-sample type is: sequencing the barcode label in each short sequence of the sample of the single-barcode multi-sample type, and sequencing the gene sequence in each short sequence of the sample of the single-barcode multi-sample type after the sequencing of the barcode label is completed. Specifically, a single-end sequencing process includes: DNB loading -> loading prime -> postloading -> barcode pre-treatment (barcode prime) -> barcode sequencing -> sequence prime -> sequence prime cleaning-> read1 (first DNB) sequencing. A paired-end sequencing process includes: DNB loading -> loading prime -> postloading -> barcode prime -> barcode sequencing -> sequence prime -> sequence prime clearing -> read1 (first DNB) sequencing -> PE synthesis -> read2 (first DNB) sequencing. The specific process of each of the above sub-processes is the same as that of the related art, which will not be described in detail herein.

The sequencing sequence corresponding to the one-strand-dual-barcode multi-sample type is: separately sequencing the barcode labels in each short sequence of the sample of the one-strand-dual-barcode multi-sample type; and sequencing the gene sequence in each short sequence of the sample of the one-strand-dual-barcode multi-sample type after the sequencing of the barcode labels is completed. Specifically, a single-end sequencing process includes: DNB loading -> loading prime -> postloading -> barcode1 pre-treatment -> barcode1 sequencing -> barcode2 pre-treatment -> barcode2 sequencing -> sequence prime -> sequence prime cleaning -> read1 (first DNB) sequencing. A paired-end sequencing process includes: DNB loading -> loading prime -> postloading -> barcode1 pre-treatment -> barcode 1 sequencing -> barcode2 pre-treatment -> barcode2 sequencing -> sequence prime -> sequence prime clearing -> read 1 (first DNB) sequencing -> PE synthesis -> read2 (first DNB) sequencing. The specific process of each of the above sub-processes is the same as that of the related art, which will not be described in detail herein.

The sequencing sequence corresponding to the dual-strand-dual-barcode multi-sample type is: sequencing a first barcode label in each short sequence of the sample of the dual-strand-dual-barcode multi-sample type; sequencing the gene sequence in each short sequence of the sample of the dual-strand-dual-barcode multi-sample type after the sequencing of the first barcode label is completed; and sequencing a second barcode label in each short sequence of the sample of the dual-strand-dual-barcode multi-sample type after the sequencing of the gene sequence is completed. Specifically, a single-end sequencing process includes: DNB loading -> loading prime -> postloading -> barcode1 pre-treatment -> barcode1 sequencing -> sequence prime -> sequence prime cleaning -> read1 (first DNB) sequencing -> barcode2 pre-treatment -> barcode2 sequencing. A paired-end sequencing process includes: DNB loading -> loading prime -> postloading -> barcode1 pre-treatment -> barcode 1 sequencing -> sequence prime -> sequence prime cleaning -> read1 (first DNB) sequencing -> PE synthesis -> read2 (first DNB) sequencing -> barcode2 pre-treatment -> barcode2 sequencing. The specific process of each of the above sub-processes is the same as that of the related art, which will not be described in detail herein.

In another possible implementation of the present disclosure, considering that the first detection of the barcode label may occupy the primers used for the PE synthesis, thereby affecting the sequencing quality, in order to first sequence the barcode label without affecting the sequencing quality, in the present embodiment, barcode primers having a smaller length than the historical barcode primers can be used to sequence each short sequence of any one sample of multi-sample types, and thus the barcode primers can be eluted before the PE synthesis.

The historical barcode primers refer to the barcode primers used in the existing gene sequencing technologies. It can be understood by a person skilled in the art that the historical barcode primers are typically 32 bp in length.

Optionally, in an embodiment of the present disclosure, the barcode primers used to sequence each short sequence of any one sample of the multi-sample types may be 25 bp in length. It should be noted that the length of 25 bp is merely an example and is not a limitation of the present disclosure.

In still another possible implementation of the present disclosure, in order to prevent the DNB structure from being loose when the barcode label is first detected, the nanoball structure stabilization information, xlinker, may be added before barcode primers for stabilizing the structure of DNB. The optimized sequencing quality has little difference from conventional sequencing.

In yet another possible implementation of the present disclosure, when the gene sample contains a plurality of samples, the plurality of samples are required to be sequenced together, and thus the obtained intermediate stage sequencing result data of each short sequence in each sample is required to be split based on barcode labels.

Therefore, optionally, if the gene sample includes a plurality of samples, the process of "sending the intermediate stage sequencing result data of each short sequence in each sample to a target server" in step S104 may include: splitting and classifying, based on the barcode labels corresponding to the plurality of samples, the intermediate stage sequencing result data of each short sequence in the plurality of samples, to obtain the intermediate stage sequencing result data corresponding to the plurality of samples; and sending the intermediate stage sequencing result data corresponding to the plurality of samples to the target server.

It should be noted that the above "splitting and classifying, based on the barcode labels corresponding to the plurality of samples, the intermediate stage sequencing result data of each short sequence in the plurality of samples" specifically refers to classifying the intermediate stage sequencing result data of the respective samples together by splitting and classifying the intermediate stage sequencing result data of the short sequence in the respective sample based on the barcode label corresponding to each sample.

More specifically, the intermediate stage sequencing result data of the sample of the single-barcode multi-sample type can be split and classified based on the single barcode label at the intermediate stage of sequencing; the intermediate stage sequencing result data of the sample of the one-strand-dual-barcode multi-sample type can be split and classified based on the dual barcode labels at the intermediate stage of sequencing; the intermediate stage sequencing result data of the sample of the dual-strand-dual-barcode multi-sample type can be split and classified based on the first barcode (i.e., a barcode label located before the gene sequence) at the intermediate stage of sequencing (split and classified based on the dual barcode labels at the end of sequencing).

In yet another possible implementation of the present disclosure, in the present embodiment, sequencing may be continued while generating the intermediate stage detection report, so that a complete detection report may be obtained upon the completion of sequencing.

Specifically, in an embodiment of the present disclosure, the method may further include: completely sequencing, for each of the sample in the gene sample, each short sequence of the sample based on the sequencing sequence corresponding to the sample type of the sample, to obtain complete sequencing result data of each short sequence in the sample; and sending, to the target server, the complete sequencing result data of each short sequence in each sample, and performing, by the target server, data analysis on the complete sequencing result data of each short sequence in each sample, to obtain a complete detection report.

In combination with the sequencing sequences introduced in the preceding embodiments, the process of "completely sequencing each short sequence of the sample based on the sequencing sequence corresponding to the sample type of the sample, to obtain complete sequencing result data of each short sequence in the sample" in the present embodiment may include: when the sample type of the sample is a barcode-absent single sample type, sequencing the gene sequence in each short sequence of the sample; when the sample type of the sample is a single-barcode multi-sample type, sequencing the barcode label in each short sequence of the sample, and after the sequencing of the barcode label in each short sequence of the sample is completed, sequencing the gene sequence in each short sequence of the sample; when the sample type of the sample is a one-strand-dual-barcode multi-sample type, separately sequencing two barcode labels in each short sequence of the sample, and after the sequencing of the two barcode labels is completed, sequencing the gene sequence in each short sequence of the sample; and when the sample type of the sample is a dual-strand-dual-barcode multi-sample type, sequencing a first barcode label in each short sequence of the sample, after the sequencing of the first barcode label is completed, sequencing the gene sequence in each short sequence of the sample, and after the sequencing of the gene sequence in each short sequence of the sample is completed, sequencing a second barcode label in each short sequence of the sample.

In summary, in the present embodiment, by performing at least one barcode label sequencing at the beginning of the entire sequencing process, partial sequencing data can be acquired in the intermediate stage of the sequencing to initiate data analysis, and an intermediate stage detection report can be obtained based on base information of the intermediate stage sequencing result data for performing preliminary identification analysis. At the same time, the sequencing is continued, and after the complete sequencing is finished, the data analysis is performed again using the complete sequencing result data to obtain a complete detection report for performing accurate identification and analysis.

In yet another possible implementation of the present disclosure, the intermediate stage detection report includes intermediate stage quality control results and intermediate stage identification results of each sample. The complete detection report includes complete quality control results, complete identification results, complete assembly results, and complete source-tracing results of each sample. The intermediate stage quality control results and the complete quality control results of one sample are both used to reflect the short sequences of the one sample that have a higher quality than a preset quality threshold. The intermediate stage identification results and the complete identification results of one sample are used to reflect a pathogen concentration information of the one sample. The complete assembly results of one sample are used to reflect a recombinant sample assembled from all the short sequences of the one sample. The complete source-tracing results of one sample are used to reflect a subtype of the one sample.

Specifically, for each sample included in the gene sample, the process of performing, by the target server, the data analysis based on the sequencing result data of each short sequence in the sample includes four stages, i.e., a quality control stage, an identification stage, an assembly stage, and a tracing stage.

The quality control stage refers to a stage of determining whether the quality of each short sequence is above the preset quality threshold and filtering out the short sequences with the quality below the preset quality threshold; the identification stage refers to a stage of comparing the sequencing result data of each short sequence in the sample with known pathogen sequence databases to determine pathogen concentration information of the sample; the assembly stage refers to a stage of splicing the sequencing result data of all the short sequences included in the sample into a long sequence fragment; and the tracing stage refers to a stage of comparing the spliced long sequence fragment with samples of known subtypes in databases from different countries and regions to determine the subtype of the sample.

In the present embodiment, both the intermediate stage sequencing and the complete sequencing can be analyzed according to the above four stages. Preferably, only quality control and identification can be performed during the intermediate stage sequencing to reduce the intermediate detection time.

Optionally, considering that the intermediate stage analysis results may be inaccurate, only the intermediate stage quality control results and intermediate stage identification results of each sample may be shown in the intermediate stage detection report, while the complete quality control results, the complete identification results, the complete assembly results, and the complete source-tracing results of each sample may be shown in the complete detection report, in order to avoid misleading the customer due to inaccurate analysis results.

In yet another possible implementation of the present disclosure, if the preset read length includes a first read length and a second read length greater than the first read length, the intermediate stage detection report at the first read length refers to a detection report obtained by analyzing the intermediate stage sequencing result data of each short sequence of each sample at the first read length, and the intermediate stage detection report at the second read length and the complete detection report both refer to a detection report obtained by analyzing the intermediate stage sequencing result data of short sequences identified as non-host sequences in each sample at the second read length.

Specifically, after obtaining the intermediate stage sequencing result data of each short sequence of each sample at the first read length, the target server can determine whether each short sequence of the sample belongs to a pathogenic sequence or a host sequence (e.g., a human-derived sample sequence, an animal sample sequence, etc.) or an unidentified sequence through analysis. At this moment, the detection report obtained through analysis is the intermediate stage detection report at the first read length.

After obtaining the intermediate stage sequencing result data of each short sequence of each sample at the second read length, the target server can analyze the intermediate stage sequencing result data of short sequences identified as non-host sequences in each sample at the second read length to obtain the intermediate stage detection report at the second read length.

The non-host sequences include pathogenic and unidentified sequences.

For example, upon sequencing to SE40, the target server can analyze the intermediate stage sequencing result data of each short sequence of each sample at 40 bp to determine which short sequences in the sample are pathogenic sequences and which short sequences are host sequences or unidentified sequences (optionally, each short sequence has a corresponding sequence serial number, through which some short sequences are denoted as pathogenic sequences and some short sequences are denoted as host sequences or unidentified sequences). Upon subsequent sequencing to SE100 and PE100, the intermediate stage sequencing result data of short sequences identified as non-host sequences in each sample at the second read length can be analyzed to improve analysis efficiency.

Optionally, in the present embodiment, a supplementary analysis may be further performed on the complete sequencing result data of short sequences identified as host sequences in each sample to allow the analysis to be more complete.

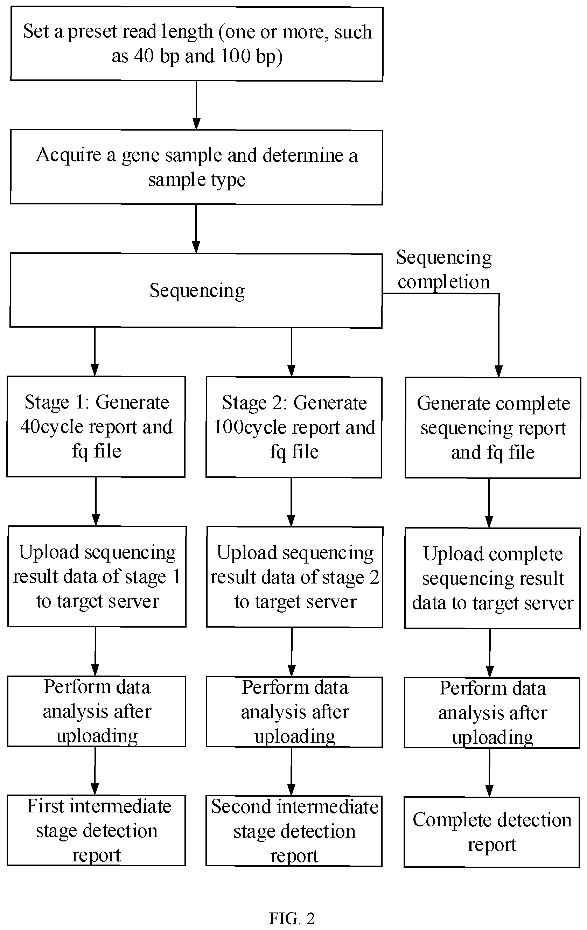

In order to enable a person skilled in the art to understand the present disclosure better, referring to FIG. 2, a schematic diagram of an analysis by sequencing application process according to an embodiment of the present disclosure is illustrated. In the present embodiment, the cycle numbers required to generate a report, i.e., a preset read length, such as 40 bp and 100 bp, can be set, a gene sample is then acquired, and a sample type of each sample included in the gene sample is determined. Thereafter, each short sequence of each sample can be sequenced based on the sequencing sequence corresponding to the sample type to which each sample belongs.

Upon the gene sequence in each short sequence of each sample is sequenced to 40 bp, the intermediate stage sequencing result data of each short sequence in each sample at stage 1 is obtained, and a 40cycle report and an fq file are generated (fq is a main result file generated by sequencing, and the full name is FASTQ file, which contains the sequencing result data and the corresponding quality value) based on the intermediate stage sequencing result data of each short sequence in each sample at stage 1. Upon the gene sequence in each short sequence of each sample is sequenced to 100 bp, the intermediate stage sequencing result data of each short sequence in each sample at stage 2 is obtained, a 100cycle report and an fq file are generated based on the intermediate stage sequencing result data of each short sequence in each sample at stage 2. Upon the completion of sequencing, a complete sequencing report and an fq file are obtained.

The sequencing result data obtained in the above three stages can be uploaded to the target server for data analysis to obtain detection reports corresponding to the three stages. Among the detection reports of the three stages, the complete detection report has the highest accuracy, followed by the second intermediate stage detection report obtained at stage 2, and the first intermediate stage detection report obtained at stage 1 has the lowest accuracy.

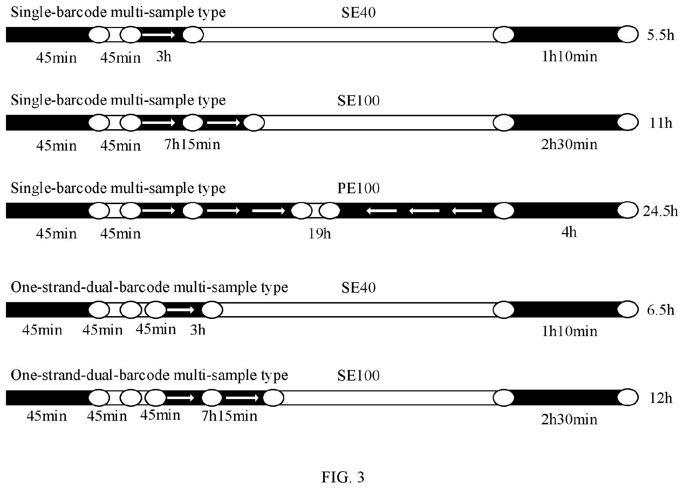

FIG. 3 is a schematic diagram illustrating the time periods for sequencing samples of multi-sample types to obtain the detection reports of the three stages. When the sample of the single-barcode multi-sample type is sequenced, an intermediate stage detection report is obtained at the 40-th cycle of the sequencing process, and the time period for obtaining the intermediate stage detection report is 5.5 hours (h); an intermediate stage detection report is obtained at the 100-th cycle, and the time period for obtaining the intermediate stage detection report is 11 hours; and a complete detection report is obtained for complete PE100 sequencing, and the time period for obtaining the complete detection report is 24.5 hours. When the sample of the one-strand-dual-barcode multi-sample type is sequenced, the time periods for obtaining the detection reports of the three stages are 6.5 hours for 40 cycles, 12 hours for 100 cycles, and 25.5 hours for complete PE100 sequencing (not shown in FIG. 3), respectively. When the sample of the dual-strand-dual-barcode multi-sample type (the time periods thereof are similar as the single-barcode multi-sample type, not shown in FIG. 3) is sequenced, the time periods for obtaining the detection reports of the three stages are 5.5 hours for 40 cycles, 11 hours for 100 cycles, and 24.5 hours for complete PE100 sequencing, respectively.

While the preceding embodiments of the method have been described as a series of actions for purposes of simplicity of description, it will be known by a person skilled in the art that the present disclosure is not limited by the order of actions described, as some steps may occur in other orders or simultaneously according to the present disclosure. In addition, a person skilled in the art would also know that the embodiments described in the specification are all preferred embodiments, and the acts and modules involved are not necessarily required by the present disclosure.

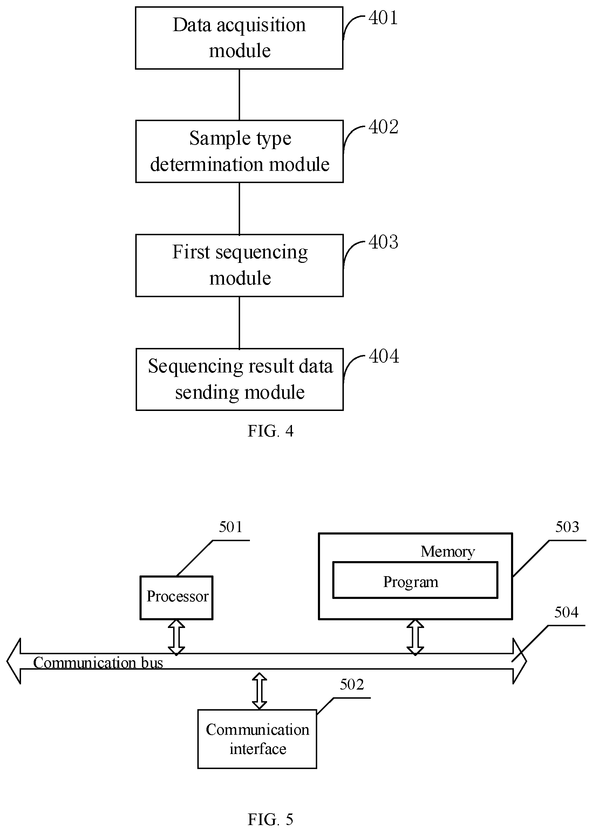

FIG. 4 is a schematic structural diagram of a gene sequencing apparatus according to an embodiment of the present disclosure, corresponding to the gene sequencing method according to an embodiment of the present disclosure described in FIG. 1. The gene sequencing apparatus described in the present embodiment can be specifically applied to a gene sequencer in practical applications. The apparatus can include a data acquisition module 401, a sample type determination module 402, a first sequencing module 403, and a sequencing result data sending module 404.

The data acquisition module 401 is configured to acquire a gene sample to be detected and a preset read length. The gene sample includes at least one sample, any one sample of the at least one sample comprising at least one short sequence. Each short sequence includes a gene sequence to be detected and at most two barcode labels. In the case that a short sequence comprises at least one barcode label, the at least one barcode label in the short sequence being located upstream of the gene sequence.

The sample type determination module 402 is configured to determine a sample type of each of the at least one sample comprised in the gene sample.

The first sequencing module 403 is configured to sequence, for each of the at least one sample in the gene sample, each short sequence of the sample based on a sequencing sequence corresponding to the sample type of the sample, until the gene sequence in each short sequence of the sample is sequenced to the preset read length, to obtain intermediate stage sequencing result data of each short sequence in the sample.

The sequencing result data sending module 404 is configured to send, to a target server, the intermediate stage sequencing result data of each short sequence in each of the at least one sample, enabling the target server to perform data analysis on the intermediate stage sequencing result data of each short sequence in each of the at least one sample, to obtain an intermediate stage detection report.

In a possible implementation, the above sample type determination module 402 may be specifically configured to: in the case that the gene sample includes one sample, determine that the sample belongs to a barcode-absent single sample type; and in the case that the gene sample includes a plurality of samples, for each sample in the plurality of samples, determine, when a short sequence of the sample comprises one barcode label, that the sample belongs to a single-barcode multi-sample type; determine, when the short sequence of the sample comprises two barcode labels located in the same strand, that the sample belongs to a one-strand-dual-barcode multi-sample type; and determine, when the short sequence of the sample comprises two barcode labels located in two strands, that the sample belongs to a dual-strand-dual-barcode multi-sample type.

In a possible implementation, the sequencing sequence corresponding to the above barcode-absent single sample type is sequencing the gene sequence in each short sequence of the sample of the barcode-absent single sample type.

In a possible implementation, the sequencing sequence corresponding to the above single-barcode multi-sample type is: sequencing the barcode label in each short sequence of the sample of the single-barcode multi-sample type; and sequencing, subsequent to the completion of said sequencing the barcode label, the gene sequence in each short sequence of the sample of the single-barcode multi-sample type.

In a possible implementation, the sequencing sequence corresponding to the above one-strand-dual-barcode multi-sample type is: sequencing the two barcode labels in each short sequence of the sample of the one-strand-dual-barcode multi-sample type, separately; and sequencing, subsequent to the completion of said sequencing the two barcode labels, the gene sequence in each short sequence of the sample of the one-strand-dual-barcode multi-sample type.

In a possible implementation, the sequencing sequence corresponding to the above dual-strand-dual-barcode multi-sample type is: sequencing a first barcode label in each short sequence of the sample of the dual-strand-dual-barcode multi-sample type; sequencing, subsequent to the completion of said sequencing the first barcode label, the gene sequence in each short sequence of the sample of the dual-strand-dual-barcode multi-sample type; and sequencing, subsequent to the completion of said sequencing the gene sequence, a second barcode label in each short sequence of the sample of the dual-strand-dual-barcode multi-sample type.

In a possible implementation, the barcode primers used to sequence each short sequence of any one sample of the multi-sample types have a smaller length than historical barcode primers; the multi-sample types include the single-barcode multi-sample type, the one-strand-dual-barcode multi-sample type, and the dual-strand-dual-barcode multi-sample type.

In a possible implementation, the preset read length includes at least one read length.

In a possible implementation, when the gene sample includes a plurality of samples, the above sequencing result data sending module 404 may specifically configured to: split and classify based on barcode labels corresponding to the plurality of samples, the intermediate stage sequencing result data of each short sequence in the plurality of samples, to obtain the intermediate stage sequencing result data corresponding to the plurality of samples; and send the intermediate stage sequencing result data corresponding to the plurality of samples to the target server.

In a possible implementation, the gene sequencing apparatus provided in the embodiment of the present disclosure may further include a second sequencing module and a complete sequencing result data sending module.

The second sequencing module is configured to completely sequence, for each sample in the gene sample, each short sequence of the sample based on the sequencing sequence corresponding to the sample type of the sample, to obtain complete sequencing result data of each short sequence in the sample.

The complete sequencing result data sending module is configured to send the complete sequencing result data of each short sequence in each sample to the target server, enabling the target server to perform data analysis on the complete sequencing result data of each short sequence in each sample to obtain a complete detection report.

In a possible implementation, the intermediate stage detection report includes intermediate stage quality control results and intermediate stage identification results of each sample. The complete detection report includes complete quality control results, complete identification results, complete assembly results, and complete source-tracing results of each sample. The intermediate stage quality control results and the complete quality control results of a sample are both used to reflect the short sequences of the one sample that have a higher quality than a preset quality threshold. The intermediate stage identification results and the complete identification results of one sample are used to reflect a pathogen concentration information of the one sample. The complete assembly results of one sample are used to reflect a recombinant sample assembled from all the short sequences of the one sample. The complete source-tracing results of one sample are used to reflect a subtype of the one sample.

In a possible implementation, when the preset read length includes a first read length and a second read length that is greater than the first read length, the intermediate stage detection report at the first read length refers to a detection report obtained by analyzing the intermediate stage sequencing result data of each short sequence of each sample at the first read length, and the intermediate stage detection report at the second read length and the complete detection report both refer to a detection report obtained by analyzing the intermediate stage sequencing result data of short sequences identified as non-host sequences in each sample at the second read length.

The embodiments of the present disclosure further provide a gene sequencing device. Optionally, FIG. 5 is a block diagram of a hardware architecture of a gene sequencing device. With reference to FIG. 5, the hardware architecture of the gene sequencing device may include at least one processor 501, at least one communication interface 502, at least one memory 503, and at least one communication bus 504.

In the embodiment of the present disclosure, the number of the processor 501, the communication interface 502, the memory 503, and the communication bus 504 are at least one, and the processor 501, the communication interface 502, and the memory 503 communicate with each other via the communication bus 504.

The processor 501 may be a central processing unit (CPU), an application-specific integrated circuit (ASIC), or one or more integrated circuits configured to implement the embodiments of the present disclosure, etc.

The memory 503 may include high-speed random access memory (RAM) or non-volatile memory, such as at least one disk memory.

The memory 503 has a program stored thereon. The processor 501 may call the program stored in the memory 503 to: acquire a gene sample to be detected and a preset read length, the gene sample including at least one sample, any one sample of the at least one sample including at least one short sequence, each short sequence including a gene sequence to be detected and at most two barcode labels, and in the case that a short sequence comprises at least one barcode label, the at least one barcode label in the short sequence being located upstream of the gene sequence; determine a sample type of each sample included in the gene sample; sequence, for each of the at least one sample in the gene sample, each short sequence of the sample based on a sequencing sequence corresponding to the sample type of the sample, until the gene sequence in each short sequence of the sample is sequenced to the preset read length, to obtain intermediate stage sequencing result data of each short sequence in the sample; and send, to a target server, the intermediate stage sequencing result data of each short sequence in each of the at least one sample, and perform, by the target server, data analysis on the intermediate stage sequencing result data of each short sequence in each of the at least one sample, to obtain an intermediate stage detection report.

Optionally, the refinement functions and extended functions of the program may refer to the above description.

The embodiments of the present disclosure further provide a readable storage medium having a computer program stored thereon. The computer program, when executed by a processor, implements the gene sequencing method as described above.

Optionally, the refinement functions and extended functions of the program may refer to the above description.

Finally, it should be noted that in this specification, relational terms such as first and second are used only to differentiate an entity or operation from another entity or operation and do not necessarily require or imply that any actual relationship or sequence exists between these entities or operations. Moreover, the terms "comprise," "include," "contain," or any other variation thereof are intended to encompass a non-exclusive inclusion, so that a process, method, article, or device that includes a list of elements not only includes those elements but also includes other elements that are not expressly listed, or further includes elements inherent to such process, method, article, or device. Without more restrictions, the elements defined by the sentence "including a ..." do not exclude the existence of other identical elements in the process, method, article, or device, including the elements.

The respective embodiments of the present specification are described in a progressive manner. Each embodiment focuses on the difference from other embodiments, and the same and similar parts between the respective embodiments may refer to each other.

The above description of the disclosed embodiments is provided to enable a person skilled in the art to implement or practice the present disclosure. Various modifications to these embodiments will be readily apparent to a person skilled in the art, and the generic principles defined herein may be implemented in other embodiments without departing from the spirit or scope of the present disclosure. Thus, the present disclosure is not intended to be limited to the embodiments shown herein but falls within the widest scope consistent with the principles and novel features disclosed herein.

## Claims

1. A gene sequencing method, comprising:
acquiring a gene sample to be detected and a preset read length, wherein the gene sample comprises at least one sample, any one sample of the at least one sample comprising at least one short sequence, each short sequence comprising a gene sequence to be detected and at most two barcode labels, and in the case that a short sequence comprises at least one barcode label, the at least one barcode label in the short sequence being located upstream of the gene sequence;
determining a sample type of each of the at least one sample comprised in the gene sample;
sequencing, for each of the at least one sample in the gene sample, each short sequence of the sample based on a sequencing sequence corresponding to the sample type of the sample, until the gene sequence in each short sequence of the sample is sequenced to the preset read length, to obtain intermediate stage sequencing result data of each short sequence in the sample; and
sending, to a target server, the intermediate stage sequencing result data of each short sequence in each of the at least one sample, and performing, by the target server, data analysis on the intermediate stage sequencing result data of each short sequence in each of the at least one sample, to obtain an intermediate stage detection report.

2. The gene sequencing method according to claim 1, wherein said determining the sample type of each of the at least one sample comprised in the gene sample comprises:
in the case that the gene sample comprises one sample, determining that the one sample belongs to a barcode-absent single sample type; and
in the case that the gene sample comprises a plurality of samples, for each sample of the plurality of samples,
determining, when a short sequence of the sample comprises one barcode label, that the sample belongs to a single-barcode multi-sample type;
determining, when the short sequence of the sample comprises two barcode labels located in the same strand, that the sample belongs to a one-strand-dual-barcode multi-sample type; and
determining, when the short sequence of the sample comprises two barcode labels located in two strands, that the sample belongs to a dual-strand-dual-barcode multi-sample type.

3. The gene sequencing method according to claim 2, wherein:
a sequencing sequence corresponding to the barcode-absent single sample type is: sequencing the gene sequence in each short sequence of the sample of the barcode-absent single sample type;
a sequencing sequence corresponding to the single-barcode multi-sample type is: sequencing the barcode label in each short sequence of the sample of the single-barcode multi-sample type; and sequencing, subsequent to the completion of said sequencing the barcode label, the gene sequence in each short sequence of the sample of the single-barcode multi-sample type;
a sequencing sequence corresponding to the one-strand-dual-barcode multi-sample type is: sequencing the two barcode labels in each short sequence of the sample of the one-strand-dual-barcode multi-sample type, separately; and sequencing, subsequent to the completion of said sequencing the two barcode labels, the gene sequence in each short sequence of the sample of the one-strand-dual-barcode multi-sample type; and
a sequencing sequence corresponding to the dual-strand-dual-barcode multi-sample type is: sequencing a first barcode label in each short sequence of the sample of the dual-strand-dual-barcode multi-sample type; sequencing, subsequent to the completion of said sequencing the first barcode label, the gene sequence in each short sequence of the sample of the dual-strand-dual-barcode multi-sample type; and sequencing, subsequent to the completion of said sequencing the gene sequence, a second barcode label in each short sequence of the sample of the dual-strand-dual-barcode multi-sample type.

4. The gene sequencing method according to claim 3, wherein barcode primers used to sequence each short sequence of any one sample of multi-sample types have a smaller length than historical barcode primers, wherein the multi-sample types comprise the single-barcode multi-sample type, the one-strand-dual-barcode multi-sample type, and the dual-strand-dual-barcode multi-sample type.

5. The gene sequencing method according to claim 1, wherein the preset read length comprises at least one read length.

6. The gene sequencing method according to claim 1, wherein when the gene sample comprises a plurality of samples, said sending, to the target server, the intermediate stage sequencing result data of each short sequence in each of the plurality of samples comprises:
splitting and classifying, based on barcode labels corresponding to the plurality of samples, the intermediate stage sequencing result data of each short sequence in the plurality of samples, to obtain the intermediate stage sequencing result data corresponding to the plurality of samples; and
sending the intermediate stage sequencing result data corresponding to the plurality of samples to the target server.

7. The gene sequencing method according to claim 1, further comprising:
completely sequencing, for each of the at least one sample in the gene sample, each short sequence of the sample based on the sequencing sequence corresponding to the sample type of the sample, to obtain complete sequencing result data of each short sequence in the sample; and
sending, to the target server, the complete sequencing result data of each short sequence in each sample, and performing, by the target server, data analysis on the complete sequencing result data of each short sequence in each sample, to obtain a complete detection report.

8. The gene sequence method according to claim 7, wherein:
the intermediate stage detection report comprises intermediate stage quality control results and intermediate stage identification results of each sample;
the complete detection report comprises complete quality control results, complete identification results, complete assembly results, and complete source-tracing results of each sample;
the intermediate stage quality control results and the complete quality control results of one sample are used to reflect the short sequences of the one sample that have a higher quality than a preset quality threshold;
the intermediate stage identification results and the complete identification results of one sample are used to reflect a pathogen concentration information of the one sample;
the complete assembly results of one sample are used to reflect a recombinant sample assembled from all the short sequences of the one sample; and
the complete source-tracing results of one sample are used to reflect a subtype of the one sample.

9. The gene sequencing method according to claim 7, wherein, when the preset read length comprises a first read length and a second read length greater than the first read length:
an intermediate stage detection report at the first read length refers to a detection report obtained by analyzing the intermediate stage sequencing result data of each short sequence of each sample at the first read length; and
an intermediate stage detection report at the second read length and the complete detection report both refer to a detection report obtained by analyzing the intermediate stage sequencing result data of short sequences identified as non-host sequences in each sample at the second read length.

10. A gene sequencing apparatus, comprising
a data acquisition module configured to acquire a gene sample to be detected and a preset read length, wherein the gene sample comprises at least one sample, any one sample of the at least one sample comprising at least one short sequence, each short sequence comprising a gene sequence to be detected and at most two barcode labels, and in the case that a short sequence comprises at least one barcode label, the at least one barcode label in the short sequence being located upstream of the gene sequence;
a sample type determination module configured to determine a sample type of each of the at least one sample comprised in the gene sample;
a first sequencing module configured to sequence, for each of the at least one sample in the gene sample, each short sequence of the sample based on a sequencing sequence corresponding to the sample type of the sample, until the gene sequence in each short sequence of the sample is sequenced to the preset read length, to obtain intermediate stage sequencing result data of each short sequence in the sample; and
a sequencing result data sending module configured to send, to a target server, the intermediate stage sequencing result data of each short sequence in each of the at least one sample, enabling the target server to perform data analysis on the intermediate stage sequencing result data of each short sequence in each of the at least one sample, to obtain an intermediate stage detection report.

11. A gene sequencing device, comprising:
a memory having a program stored thereon; and
a processor configured to execute the program to implement respective steps of the gene sequencing method according to any one of claims 1 to 9.

12. A readable storage medium, having a computer program stored thereon, wherein the computer program, when executed by a processor, implements respective steps of the gene sequencing method according to any one of claims 1 to 9.
